# EUROPEAN PATENT SPECIFICATION

(11) **EP 4 081 785 B1**
(45) Date of publication and mention of the grant of the patent: **09.07.2025**
(21) Application number: 20906030.0
(22) Date of filing: 04.12.2020
(51) Int. Cl.: G01N 21/64, A61M 25/06, A61M 25/01

(54) **REAL TIME FLUORESCENT DETECTION SYSTEMS FOR MEDICAL DEVICES**
ECHTZEIT-FLUORESZENZDETEKTIONSSYSTEME FÜR MEDIZINPRODUKTE
SYSTÈMES DE DÉTECTION PAR FLUORESCENCE EN TEMPS RÉEL DESTINÉ À DES DISPOSITIFS MÉDICAUX

(30) Priority: 27.12.2019 US 201962954404 P
(43) Date of publication of application: 02.11.2022
(73) Proprietor: Acies Medical LLC, Saint Paul MN 55105 (US)
(72) Inventor: SIPPLE, Daniel, Saint Paul, MN 55105 (US); NORLING, Brian, L., Santa Barbara, CA 93109 (US)
(74) Representative: FRKelly
(86) International application number: PCT/US2020/063181
(87) International publication number: WO 2021/133533

(56) References cited:
- US-A1- 2016 310 704
- US-A1- 2017 173 275
- US-A1- 2017 311 807
- US-A1- 2018 008 172
- US-B1- 6 246 901
- US-B2- 10 342 416
- US-B2- 10 470 712

## Description

### CROSS-REFERENCE TO RELATED APPLICATIONS

This application claims the benefit of U.S. Provisional Patent Application Serial No. 62/954,404, currently pending and titled, "REAL TIME FLUORESCENT DETECTION SYSTEMS FOR MEDICAL DEVICES", which was filed December 27, 2019.

### FIELD

The present invention relates to real time detection of biological substances and distinguishing between bodily tissues.

### BACKGROUND

Efforts to improve surgical outcomes and cost structure, particularly with spinal surgery, have led to increased use of minimally invasive procedures. These procedures often use image-guided modalities such as fluoroscopy, CT, nerve stimulators, and, more recently, Doppler ultrasound. While often involving less risk than surgery, minimally invasive spinal procedures, pain management procedures, nerve blocks, ultrasound guided interventions, biopsy, and percutaneous placement or open intra-operative placement continue to carry risks of ineffective outcome and iatrogenic injuries, such as infection, stroke, paralysis and death due to penetration of various structures including, but not limited to, organs, soft tissues, vascular structures, and neural tissue such as, catastrophically, the spinal cord. Injuries can occur regardless of practitioner experience because a surgical instrument must proceed through several layers of bodily tissues and fluids to reach the desired space in the spinal canal.

To illustrate, the intrathecal (or subarachnoid) space of the spinal region, where many medications are administered, houses nerve roots and cerebrospinal fluid (CSF) and lays between two of the three membranes that envelope the central nervous system. The outermost membrane of the central nervous system is the dura mater, the second is the arachnoid mater, and the third, and innermost membrane, is the pia mater. The intrathecal space is in between the arachnoid mater and the pia mater. To get to this area, a surgical instrument may need to first get through skin layers, fat layers, the interspinal ligament, the ligamentum flavum, the epidural space, the dura mater, the subdural space, and the intrathecal space. Additionally, in the case of a needle used to administer medication, the entire needle opening must be within the sub-arachnoid space. Because of the complexities involved in inserting a surgical instrument into the intrathecal space, penetration of the spinal cord and neural tissue is a known complication of minimally invasive spine procedures and spine surgery. Additionally, some procedures require the use of larger surgical instruments. For example, spinal cord stimulation, a form of minimally invasive spinal procedure wherein small wire leads can be inserted in the spinal epidural space, may require that a 14-gauge needle be introduced into the epidural space in order to thread the stimulator lead. Needles of this gauge can be technically more difficult to control, posing a higher risk of morbidity. Complications can include dural tear, spinal fluid leak, epidural vein rupture with subsequent hematoma, and direct penetration of the spinal cord or nerves with resultant paralysis. These and other high-risk situations, such as spinal interventions and radiofrequency ablation, can occur when a practitioner is unable to detect placement of the needle or surgical apparatus tip in critical anatomic structures.

At present, detection of such structures is operator dependent, wherein operators utilize tactile feel, contrast agents, anatomical landmark palpation and visualization under image-guided modalities. The safety of patients can rely upon the training and experience of the practitioner in tactile feel and interpretation of the imagery. Even though additional training and experience may help a practitioner, iatrogenic injury can occur independently of practitioner experience and skill because of anatomic variability, which can arise naturally or from repeat procedures in the form of scar tissue. Fellowship training in some procedures, such as radiofrequency ablation, may not be sufficiently rigorous to ensure competence; even with training, outcomes from the procedure can vary considerably. In the case of epidural injections and spinal surgery, variability in the thickness of the ligamentum flavum, width of the epidural space, dural ectasia, epidural lipomatosis, dural septum, and scar tissue all can add challenges to traditional verification methods even for highly experienced operators. Additionally, repeat radiofrequency procedures that are performed when nerves regenerate, often a year or more later, are often less effective and more difficult because the nerves' distribution after regeneration creates additional anatomic variability.

US2017/311807A1 describes apparatuses and methods for detecting biomarkers which include using a probe to be inserted into subcutaneous tissue to detect the presence of biomarkers.

### SUMMARY

Features and advantages of the invention will be more readily understood from the following detailed description. The present disclosure provides a probe for real-time sensing of a target biomarker as set out in claim 1. Further advantageous features are set out in the dependent claims. A biomarker detection system is disclosed that includes a target biomarker in a biological system. The disclosed biomarker detection system includes real-time fluorescent probe. The disclosed probe for real-time sensing of a target biomarker includes a needle having an opening, a fluorescent probe encased within the needle. The fluorescent probe includes a fluorescent coating disposed within and covering the opening of the needle, wherein the fluorescent coating is in contact with biological tissue. In some examples, the biological tissue includes blood. The disclosed fluorescent probe also includes an ion-consuming coating within the needle and adjacent to the fluorescent coating and a fiber optic waveguide in contact with the ion-consuming coating.

In this disclosure, the terms:
"optical receiver" refers to a light detecting device structured and configured to detect light returning along the optical path from the bioluminescent device to the optical detector;
"optical detector" refers to a device that senses and may measure the amount of light in its optical path;
"optical coupler" refers to a device that is structured and configured to couple light between a fluid channel and at least one optical fiber; and
"optical filter" refers to a device that receives light and allows only light with specific properties such as wavelength, polarity, intensity, or other selective properties to pass therethrough.

In view of these considerations, it would be desirable to provide systems and methods that provide real-time feedback to assist in the precise placement of surgical instruments into patients' anatomies.

### BRIEF DESCRIPTION OF THE DRAWINGS

The following description should be read with reference to the drawings. The drawings, which are not necessarily to scale, depict examples and are not intended to limit the scope of the disclosure. The disclosure may be more completely understood in consideration of the following description with respect to various examples in connection with the accompanying drawings, in which:
FIG. 1 is a schematic quasi-cross-sectional view of an illustrative example of a portion of a biomarker detection system according to the present disclosure;
FIG. 2 is a schematic quasi-cross-sectional view of another illustrative example of a portion of a biomarker detection system according to the present disclosure;
FIG. 3A is a schematic cross-sectional view of still another illustrative example of components of a portion of a biomarker detection system according to the present disclosure;
FIG. 3B is a schematic cross-sectional view that provides an enlarged depiction illustrating details of some of the components of the system of FIG. 3A;
FIG. 4 is a schematic cross-sectional view of yet another illustrative example of components of portion of a biomarker detection system according to the present disclosure;
FIG. 5A is a schematic cross-sectional view of still yet another illustrative example of a portion of a biomarker detection system according to the present disclosure;
FIG. 5B is a schematic cross-sectional view that provides an enlarged depiction illustrating details of some of the components of the system of FIG. 5A;
FIG. 6A is a schematic cross-sectional view of yet still another illustrative example of a portion of a biomarker detection system according to the present disclosure;
FIG. 6B is a schematic cross-sectional view that provides an enlarged depiction illustrating details of some of the components of the system of FIG. 6A;
FIG. 7A is a schematic cross-sectional view of an embodiment of a biomarker detection needle according to the present disclosure;
FIG. 7B is a schematic plan view of the needle of 7A from a viewpoint at the left of the needle as depicted in FIG. 7A;
FIGS. 8A, 8B, 8C, and 8D are schematic cross-sectional views down the bores of needles according to the present disclosure that provide optical fibers and lumens along their lengths;
FIG. 9 is a schematic cross-sectional diagram of an embodiment of a fiber optic sensor for distinguishing between air and liquid useful in a provided disclosure;
FIG. 10 is a schematic cross-sectional view down the bore of an illustrative example of a needle system that can incorporate a disclosed fiber optic air sensor;
FIG. 11 is a schematic cross-sectional view of an embodiment of a needle system that can incorporate a sonic air sensor;
FIG. 12A is a schematic plan view of an illustrative example of an embodiment of a needle system that can incorporate an electrical air sensor;
FIG. 12B is a schematic side cross-sectional view of the needle system of FIG. 12A;
FIG. 12C is a schematic cross-sectional view down the bore of the needle system of FIG. 12A;
FIG. 13A is a schematic side cross-sectional view of an illustrative example of another embodiment of a needle system that can incorporate an electrical air sensor;
FIG. 13B is a schematic cross-sectional view down the bore of one configuration of the needle system of FIG. 13A;
FIG. 13C is a schematic cross-sectional view down the bore of another configuration of the needle system of FIG. 13A;
FIG. 14 is a schematic Jablonski diagram showing possible transitions between electronic states of a molecule;
FIG. 15 is a chemical structure of fluorescein; and
FIG. 16 is planar side cross-section illustration of an embodiment of a real-time fluorescent probe.

The disclosed biomarker detection system and real-time fluorescent probe can improve upon some of the shortcomings in the present art. Its use can improve surgical outcomes and cost structure, particularly with spinal and other minimally invasive surgical procedures. The disclosed biomarker detection device can take the operator dependency out of finding target biomarker materials instead of relying on tactile feel, contrast agents, anatomical landmark palpitation, and visualization under image-guided modalities thereby improving the safety and efficacy of procedures requiring biomarker identification.

### DETAILED DESCRIPTION

The present disclosure relates to systems and methods used to detect biological substances, such as bodily fluids and tissues, including blood, and for distinguishing between bodily media, such as liquid and air. Various examples of systems and methods are to be described in detail with reference to the drawings, wherein like reference numerals may represent like parts and assemblies throughout the several views. Reference to various examples does not limit the scope of the systems and methods disclosed herein. Additionally, any examples set forth in this specification are not intended to be limiting and merely set forth some of the many possible embodiments for the systems and methods. It is understood that various omissions and substitutions of equivalents are contemplated as circumstances may suggest or render expedient, but these are intended to cover applications or embodiments without departing from the spirit or scope of the disclosure. Also, it is to be understood that the phraseology and terminology used herein are for the purpose of description and should not be regarded as limiting.

The present disclosure provides systems and methods structured, configured, and/or capable of detecting one or more biomarkers via the interaction(s) of the biomarkers with one or more detection materials, and the optical detection of said interaction(s). In some examples, the interaction of a biomarker with a detection material can result in a luminescent emission of light that can be sensed, with said sensing of luminescent light providing evidence of the interaction, and hence, the presence of the biomarker. In some of these examples, emission of light can be an intrinsic chemiluminescent product of the interaction between the biomarker and the detection material. In some others of these examples, illumination by an external light source of the detection material, when in the presence of the biomarker, can result in a fluorescent or phosphorescent emission of light that can be sensed. The present disclosure provides systems and methods that can provide detection of biomarkers via sensing of chemiluminescence, fluorescence, and/or phosphorescence.

While multiple examples of biomarker detection systems illustrated and described in the present disclosure include, or can be used in conjunction with, needles and fluid delivery systems, the applications of the disclosed biomarker detection systems and methods are not limited to fluid delivery applications. In the present disclosure, medicinal fluids can be delivered through the disclosed fluid delivery systems. Fluid delivery systems incorporating detection technologies of the present disclosure can be employed to deliver wires/leads, nanoparticles, and any suitable pharmacological or otherwise therapeutic agents, including regenerative medicines and chemotherapy drugs.

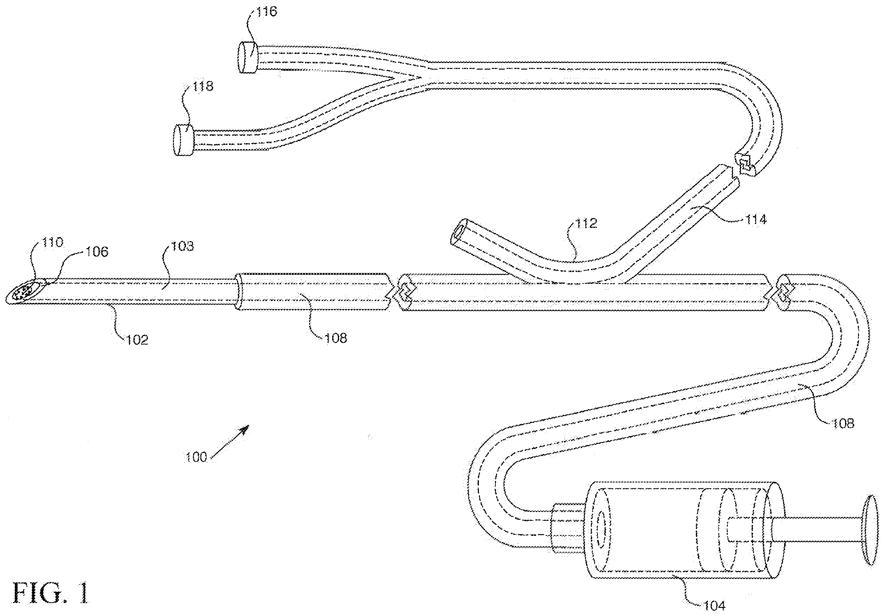

FIG. 1 schematically depicts an illustrative example of a biomarker detection system **100** of the present disclosure. System **100** can include needle **102** having lumen **103** that can deliver a fluid from syringe **104,** or other suitable fluid dispensing system, to tip **106** of the needle via fluid channel **108** (said fluid channel including lumen **103).** Biomarker luminescent material **110** can be placed at tip **106** or inside and adjacent to tip **106** of needle **102,** such as via a coating process.

System **100** can include optical coupler **112** that can be structured and configured to couple light between fluid channel **108** and optical fiber **114.** Optical coupler **112** and any other optical couplers of the present disclosure can be structured and configured to perform as a wavelength division multiplexer, which can improve signal-to-noise ratio by, for example, filtering the spectrum of light that reaches a detector. Optical fiber **114** can be selected with core material having an index of refraction that is substantially close or essentially equal to an index of refraction of a fluid in fluid channel **108,** such that light can readily be coupled between the two while minimizing reflectance losses and other optical issues that can arise from an optical mismatch. Alternatively, or in addition, the index of refraction of the fluid in channel **108** can be adjusted to substantially or essentially exactly match the index of the core of optical fiber **114,** for example, by selecting the concentration of various components of the fluid, such as glucose, alcohol, saccharide salts, and/or any other biocompatible fluid(s). Fluid channel **108** can be structured and configured such that it, with fluid present, can serve as an optical waveguide.

A light source **116** such as a diode laser or other suitable light source can be optically coupled to optical fiber **114** such that light from the light source can be delivered to tip **106** of needle **102** via the optical fiber and the optical wave guide provided by fluid channel **108,** and more specifically, to biomarker luminescent material **110** at said tip. Light that is emitted or otherwise scatters from biomarker luminescent material **110** can be returned by the optical path provided by fluid channel **108** and optical fiber **114** to an optical receiver **118,** which can be a photo detector or any other suitable light detecting device structured and configured to detect light returning along the optical path from tip **106** of needle **102.** Optical coupler **112** can be structured, configured, and tuned such that it can effectively couple light between fluid channel **108** and optical fiber 114 for any relevant optical frequencies, including one or more emission frequencies of light source 116 and the frequency(ies) of luminescence of biomarker luminescent material **110.**

Via various mechanisms, light from light source **116** can undesirably reach optical receiver **118,** such as via back-reflections from tissue and other back-scattering avenues, adding to the noise read by receiver **118** as it measures the signal of luminescent emission from biomarker luminescent material **110.** This source of measurement noise can be countered in multiple ways. As aforementioned, optical coupler **112** can be structured and configured to perform as a wavelength division multiplexer, which can selectively filter the frequencies of light incident upon optical receiver **118** to the frequency(ies) emitted from biomarker luminescent material **110.** Alternatively or in addition, optical receiver **118** can include a filter to selectively prevent frequencies other than the frequency(ies) of luminescence of biomarker luminescent material **110** from reaching the optical receiver, such as illumination light from light source **116,** and other light that may exist in the environment, such as ambient room lighting. Other measures to improve signal-to-noise can be taken, such as filtering room lighting to attenuate emission at the frequencies of sensitivity of optical receiver **118.** Such wavelength and frequency filtration/sensitivity considerations may apply to any relevant systems of the present disclosure.

Another technique that can be employed to improve signal-to-noise for detection at optical receiver **118** of light emitted from biomarker luminescent material **110** is time division multiplexing. By temporally separating the illumination of biomarker luminescent material **110** by light source 116 from the detection of luminescent emission from the material, this source of noise can be circumvented. In an illustrative example, light source **116** can be driven with a fully-on, fully-off square wave. With a light source **116** that can be switched-off sufficiently quickly (that is, fast compared to the decay time constant for luminescent emission from biomarker luminescent material **110),** data collection from optical receiver **118** can be gated to be performed only when light source **116** is off, such that no back-reflected/scattered light originating from light source **116** is recorded at optical receiver **118.** Potentially, other sources of light (as might be employed in a surgical suite, for example) that might undesirably reach optical receiver **118** could also be driven with the same waveform as light source **116,** to prevent their detection. With a sufficiently high frequency, such pulsing could be imperceptible to the human eye. These time division multiplexing methods may be advantageously employed with any compatible systems and methods of the present disclosure.

In an example method of use of system **100,** needle **102** can be advanced into a patient by a clinician, with light source **116** activated to provide illumination of biomarker luminescent material **110.** A fluid having an optically suitable index of refraction can be present in fluid channel **108** (including lumen **103).** Needle **102** can be advanced until tip **106** of the needle encounters a target biomaterial (for example, blood, although other target biomaterials are possible), upon which interaction of the target biomaterial (e.g., blood) with biomarker luminescent material **110,** in combination with illumination light from light source **116,** can result in emission of light from the biomarker luminescent material, which can be detected by optical receiver **118.** A notification system (not illustrated) operatively coupled to optical receiver **118** can inform the clinician that the target biomarker has been detected. The clinician can then position tip **106** of needle **102** in accordance with the detection of the target biomaterial and knowledge of the patient's anatomy (for example, further advancing, stopping advancing, or retracting the needle). With tip **106** of needle **102** appropriately placed, delivery of a therapeutic fluid from the fluid delivery system through fluid channel **108** can be performed.

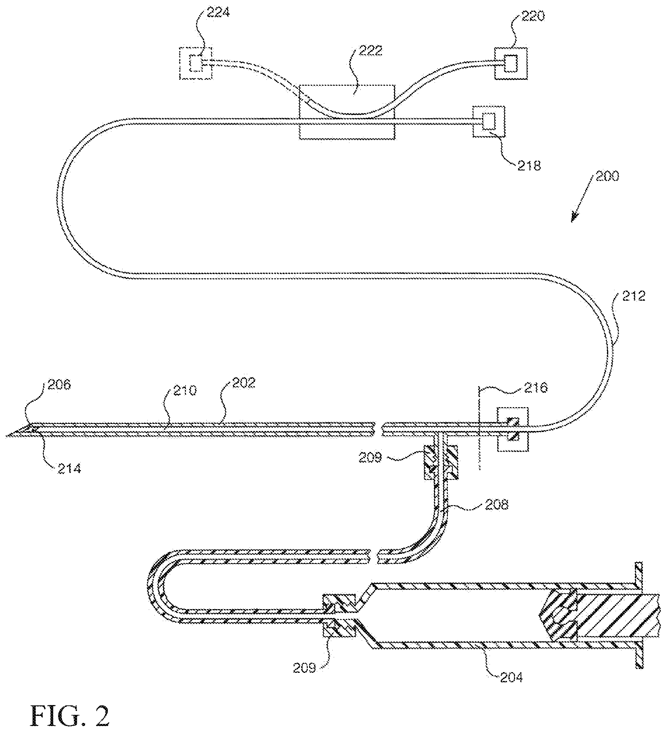

FIG. 2 schematically depicts another illustrative example of another biomarker detection system **200** of the present disclosure. System **200** can include needle **202** that can deliver a fluid from syringe **204,** or other suitable fluid dispensing system, to tip **206** of the needle via fluid channel **208.** Needle **202** and syringe **204** can be fluidically coupled via one or more fluid connectors **209,** which can be any suitable connectors, such as (but not limited to) LUER lock fittings. Fluid channel **208** can include lumen **210** of needle **202.** In a configuration of system **200** illustrated in FIG. 2, lumen **210** of needle **202** can be at least partially occupied by optical fiber **212.** Optical fiber **212** can include biomarker luminescent material **214** affixed at its distal tip, such as via a coating process. As illustrated in FIG. 2, optical fiber **212** having biomarker luminescent material **214** affixed at its distal tip can be positioned in lumen **210** of needle **202** such that the biomarker luminescent material is located at or near tip **206** of needle **202.** In some examples of such a configuration, optical fiber **212** can substantially block lumen **210** of needle **202** to the passage of fluid. In some other examples, an optical fiber present in lumen **210** may allow at least partial fluid passage. In system **200,** optical fiber **212** can be selectively retracted within lumen **210,** such that the distal tip of the fiber can be located at or near a location such as location **216,** where it may substantially not obstruct flow of fluid from syringe **204** to tip **206** of needle **202** via lumen **210.**

System **200** can include light source **218,** such as a diode laser or other suitable light source, that can be optically coupled to optical fiber **212** such that light from the light source can be delivered to the distal tip of the optical fiber, and more specifically, to biomarker luminescent material **214** at said tip. Light that is emitted or otherwise scatters from biomarker luminescent material **214** can be returned by optical fiber **212** to optical receiver **220,** which can be a photo detector or any other suitable light detecting device structured and configured to detect light returned by optical fiber **212** from the distal tip of the fiber.

System **200** can include optical coupler **222** that can be structured and configured to pass light from light source **218** to biomarker luminescent material **214** at the distal tip of optical fiber **212,** and to transmit light emitted from biomarker luminescent material **214** to optical receiver **220.** Optical coupler **222** can be tuned, for example as a wavelength division multiplexer, to selectively maximize transmission of light emitted from biomarker luminescent material **214** to optical receiver **220,** and to minimize the transmission of such emitted light back toward light source **218.** Second optical receiver **224,** which can be a photo detector or any other suitable light-detecting device, can be coupled to the optical system of system **200** via fiber optic splitter **222.** Second optical receiver **224** can be used to sense the drive signal level of emission from light source **218,** which can be used to create a differential signal for purposes of compensating for variations in intensity of light source **218** (for example, drifts due to temperature variations), when interpreting signals at optical receiver **220.** This arrangement might also be used, in some cases, to implement phase sensitive detection of the signal received at optical receiver **220** from biomarker luminescent material **214.**

System **200** can be used similarly in many aspects as described for system **100.** In operation, needle **202** of system **200** can be advanced into a patient with optical fiber **212** positioned in lumen **210** such that biomarker luminescent material **214** at the distal tip of the fiber is disposed at or near tip **206** of needle **202.** Once biomarker luminescent material **214** contacts the target biomaterial (e.g., blood), light resulting from such contact can be transmitted up the fiber to optical receiver **220** and detection indicated to a user of the system. Once the needle is properly positioned (e.g., in a bloodstream), optical fiber **212** can be retracted (for example, to 216), opening lumen **210** for the flow of fluid from syringe **204** to delivery at tip **206** of needle **202.**

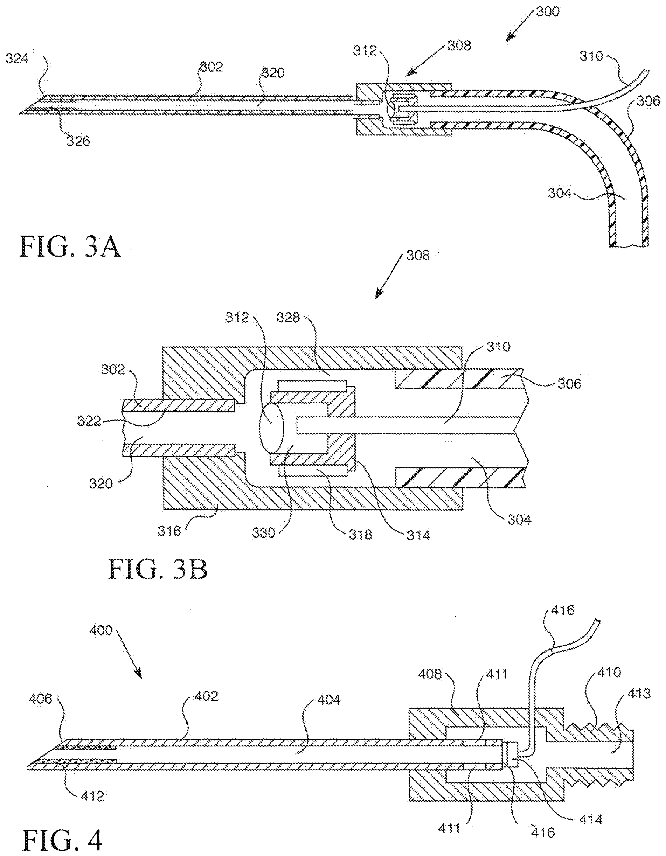

FIG. 3A schematically depicts another illustrative example of components of another biomarker detection system **300** of the present disclosure, and FIG. 3B provides an enlarged depiction that illustrates details of some of the components illustrated in FIG. 3A. System **300** can be used similarly in many aspects as described for system **100** for biomarker detection and therapeutic delivery. The system of FIGS. 3A, 3B can include needle **302** that can deliver a fluid from fluid delivery system (not shown in its entirety) via fluid channel **304** of fluid line **306.** System **300** of FIGS. 3A, 3B can include coupler **308** that can couple needle **302** with the fluid delivery system and an optical system (not shown in its entirety). System **300** of FIGS. 3A, 3B, including coupler **308,** can employ any suitable fluid connectors (not necessarily illustrated), such as (but not limited to) LUER lock fittings. The optical system can include optical fiber **310** and coupling optics **312,** which include a lens or lenses, held in optical alignment by optical mount **314.** Optical mount **314** can be held in positional relationship with respect to coupler housing **316** by support web **318.** The depicted physical arrangement of and between optical mount **314,** coupler housing **316,** and support web **318** is merely an example and should not be considered limiting.

Coupling optics **312,** when appropriately positioned and aligned with respect to optical fiber **310,** can couple or focus illumination light emitting from the optical fiber into lumen **320** of needle **302.** Interior walls **322** surrounding lumen **320** of needle **302** can be polished or otherwise smoothed, such as by an electrochemical or other suitable process, such that they can serve as a waveguide for the illumination light so coupled. In some examples, interior walls **322** surrounding lumen **320** of needle **302** can be coated or lined with a thin layer of a dielectric material such as a glass or a polymer having an index of refraction lower than the index of the fluid within the lumen, such that a total internal reflection waveguide similar to an optical fiber results, with a higher-index fluid in the lumen serving as the "core" and the lower-index thin dielectric layer coating the walls of the lumen serving as the "cladding." (This waveguide configuration can potentially be employed in any system of the present disclosure in which light propagates through a fluid channel, including in needles **102, 402, 502,** and **602** of systems **100, 400, 500,** and **600,** respectively). Illumination light, provided by any suitable light source (not illustrated) such as a diode laser, can be delivered via propagation down lumen **320** of needle **302** to its tip **324,** where biomarker luminescent material **326** can be located, such as via a coating process. Light that is emitted or otherwise scatters from biomarker luminescent material **326** can be returned by a reverse optical path (the waveguide of needle lumen **320,** then coupled by optics **312** to optical fiber **310)** to an optical receiver (not illustrated), which can be a photo detector or any other suitable light detecting device structured and configured to detect light returning by optical fiber **310** from tip **324** of needle **302.**

Coupler **308** can include one or more fluid channels **328** that can fluidically communicate between fluid channel **304** of fluid line **306** and lumen **320** of needle **302.** Optical mount **314** can define or provide void **330** that can be fluidically sealed from the fluidic path (**304, 328, 320**) of the fluid delivery system, and in which a vacuum or gas atmosphere of stable refractive index can be maintained, such that effects of index-of-refraction variations on optical coupling can be reduced or eliminated. For the same reason, the fluid-facing side of lens **312** can be a planar surface.

FIG. 4 schematically depicts another illustrative example of components of another biomarker detection system **400** of the present disclosure. System **400** of FIG. 4 can include needle **402** that can deliver a fluid from fluid delivery system (not illustrated) through lumen **404** to tip **406** of needle **402.** System **400** of FIG. 4 can include fluid coupler **408** that can couple needle **402** with the fluid delivery system via a fluid connector **410,** which can be any suitable fluid connector, such as (but not limited to) a LUER lock fitting. Coupler **408** and/or needle **402** can include or define one or more fluid channels **411** that can fluidically communicate between connector-side fluid channel **413** and lumen **404** of needle **402.**

Biomarker luminescent material **412** can be located at tip **406** of needle **402,** such as via a coating process. The interior walls surrounding lumen **404** of needle **402** can be polished or otherwise smoothed, such as by an electrochemical or other suitable process, such that they can form a waveguide to efficiently transport light emitted from biomarker luminescent material **412** to optical receiver **414,** which can be a photo detector or any other suitable light detecting device structured and configured to detect light emitted from the biomarker luminescent material. A wavelength-discriminating optical filter **416** that is tuned for one or more wavelengths of light emitted by biomarker luminescent material **412** can help reject stray light and improve signal-to-noise. Optical receiver **414** can be electrically connected to detection electronics via connection **418**

System **400** of FIG. 4 can be suited for use with biomarker luminescent material **412** that can produce light upon contact with a target biomaterial (e.g., blood) without illumination by an external light source. The omission of an illumination light source can make for a relatively simple biomarker detection device and system. One example of a biomarker luminescent material that does not necessarily require external illumination, which may be used with the system of FIG. 4, is luminol, which is discussed further elsewhere herein.

Aside from the omission of illumination of biomarker luminescent material **412** by an external light source, system **400** can be used similarly in many aspects as described for system **100** for biomarker detection and therapeutic delivery.

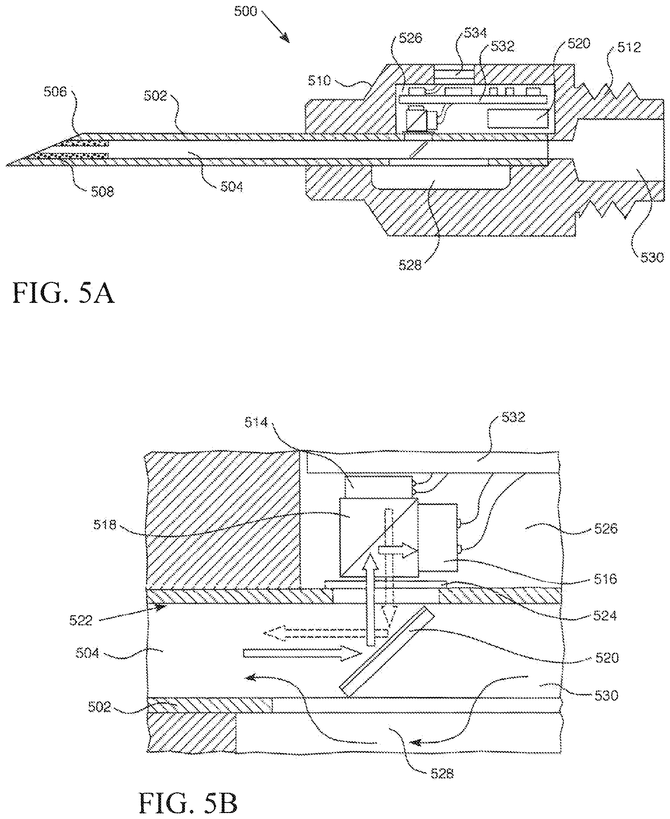

FIG. 5A schematically depicts an illustrative example of a "self-contained" biomarker detection needle system **500** of the present disclosure, and FIG. 5B provides an enlarged depiction that illustrates details of some of the components of system **500.** System **500** can include needle **502** having lumen **504** and tip **506,** at which biomarker luminescent material **508** can be located, such as via a coating process. System **500** can include mount **510** to which needle **502** can be mounted and connected to a fluid delivery system (not illustrated) via a fluid connector **512,** which can be any suitable fluid connector, such as (but not limited to) a LUER lock fitting.

Mount **510** can house optics and electronics to enable bio detection with biomarker luminescent material **508.** At mount **510,** system **500** can include light source **514** such as a diode laser or other suitable light source, and optical receiver **516,** which can be a photo detector or any other suitable light detecting device. The optical system of mount **510** can include beam splitter **518** and mirror **520.** With such an optical arrangement, illumination light from light source **514,** represented by broken-outline hollow arrows in FIG. 5B, can be directed down lumen **504** of needle **502** toward the needle's tip **506,** where it can illuminate biomarker luminescent material **508.** Light that is emitted or otherwise scatters from biomarker luminescent material **508** can be returned by a reverse optical path, as represented by solid-outline hollow arrows, to optical receiver **516.** Interior walls **522** surrounding lumen **504** of needle **502** can be polished or otherwise smoothed, such as by an electrochemical or other suitable process, such that they can serve as a waveguide for light propagating in needle **502.** The optical system of mount **510** can also include optical window **524** that can provide a barrier for fluids in lumen **504** of needle **502** from entering optical/electronic space **526** of mount **510.** Optical window **524** can be manufactured to selectively filter wavelengths (e.g., selectively passing illumination light from light source **514** and light emitted from biomarker luminescent material **508,** while blocking undesired wavelengths). Selective filtering can also be implemented at one or more surfaces of beam splitter **518.**

Mount **510** and/or needle **502** can include or define one or more fluid channels **528** that can fluidically communicate between a connector-side fluid channel **530** and lumen **504** of needle **502,** providing a fluid bypass around mirror **520.**

Light source **514** and optical receiver **516** can be electronically coupled to circuit board **532,** which can provide power and control signals to the devices and receive and process signals or other information from the devices. In FIG. 5B, both light source **514** and optical receiver **516** are illustrated as being electronically coupled to circuit board **532** via wire bonding, but this is not limiting and any suitable functional connections (such as surface mount technology) between the devices and the circuit board can be employed. The term "circuit board" as used in relation to element **532** of system **500** is used generically and should not be considered to be limiting. Circuit board **532** can include a printed circuit board with multiple discrete components, a single chip processor or "system-on-a-chip," multiple sub-boards, a hybrid system, or any other suitable arrangement capable of powering and carrying out biomarker detection system functionality with the elements of system **500.** Mount **510** can host or support any suitable user interface elements **534** which can include (but are not limited to) buttons, visual indicators such as light-emitting diodes, and audio annunciators/speakers. Circuit board **532** can include one or more wireless interfaces, such as a BLUETOOTH interface, which can incorporate any BLUETOOTH features necessary or desirable for operation, such as a detection signal, self-test, battery status, and so on. Mount **510** can house energy storage device **522** which can be a battery or any other suitable device, which can provide operational power for light source **514,** optical receiver **516,** circuit board **532** and other appropriate components hosted by mount **510.** System **500** can be used similarly in many aspects as described for system **100** for biomarker detection and therapeutic delivery.

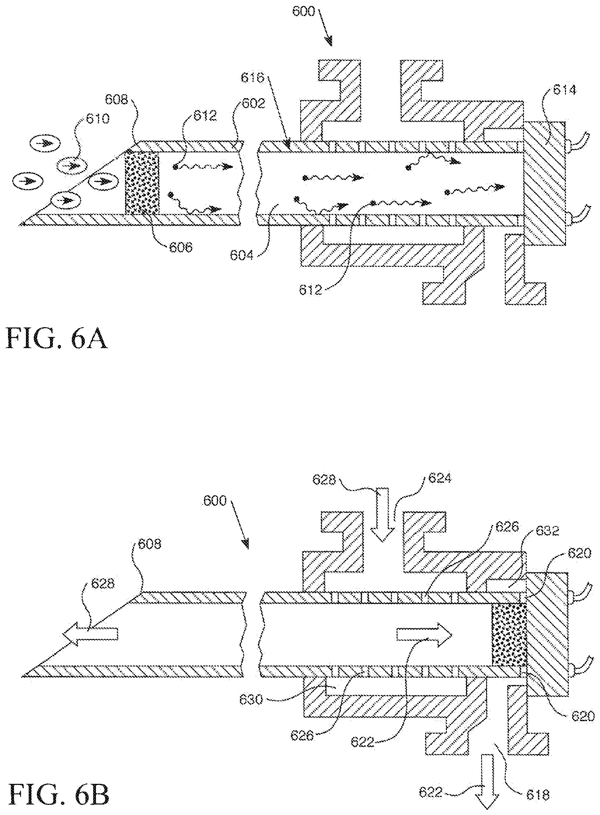

FIGS. 6A and 6B schematically depict another illustrative example of components of another biomarker detection system **600** of the present disclosure. FIG. 6A generally depicts a detection configuration of system **600** and FIG. 6B generally depicts a post-detection fluid-delivery configuration. System **600** can include needle **602** having lumen **604.** In the configuration of FIG. 6A, semi-permeable barrier **606** disposed at or near tip **608** of needle **602** can prevent a biomarker luminescent material fluid present in lumen **604** from exiting the needle at its tip, and potentially entering a patient's body. The use of semi-permeable barrier **606** in system **600** can enable the provision of a large reservoir of biomarker luminescent material fluid in lumen **604** of needle **602,** providing for greater illumination and extended duration of sensitivity as compared with other arrangements lacking such a reservoir.

Semi-permeable barrier **606** can be at least semi-permeable to target biomaterial **610,** such that the target biomaterial can come into contact with, and react with, the biomarker luminescent material fluid present in lumen **604.** In some examples, semi-permeable barrier **606** can be structured and configured such that it selectively allows passage of iron ions or iron-containing compounds, as found, for example, as a component of blood. The biomarker luminescent material fluid present in lumen **604** can be a material that is reactive with such iron ions or iron-containing compounds. In some examples, the blood stays active indefinitely. Once iron in any form present in blood contacts the biomarker luminescent material and emits radiation, the iron is typically not consumed in the reaction.

In some examples, the target biomaterial and biomarker luminescent material fluid can react such that photons **612** are generated by said reaction. As illustrated, such photons **612** could be generated in a variety of locations within lumen **604,** depending on the penetration of the target biomaterial past barrier **606** and into the volume of the lumen. In some examples, semi-permeable barrier **606** can be translucent or at least partially transparent, to allow photons **612** generated by reactions within the barrier to exit the barrier for detection.

Photons **612** produced as a result of contact between target biomaterial **610** and biomarker luminescent material fluid can propagate within lumen **604** of needle **602** to optical receiver **614,** which can be a photodetector, or any other suitable light-detecting device structured and configured to detect such light. Interior walls **616** surrounding lumen **604** of needle **602** can be polished or otherwise smoothed, such as by an electrochemical or other suitable process, such that they can serve as a waveguide for light propagating in the needle.

Where in FIG. 6A, biomarker detection system **600** is depicted in a detection configuration, in FIG. 6B the system is depicted in fluid-delivery configuration. System **600** can be reconfigured from the detection configuration of FIG. 6A to the fluid-delivery configuration of FIG. 6B following successful detection of a target biomaterial, but this is not limiting, and such a reconfiguration is not necessarily dependent upon successful biomaterial detection.

With reference to FIG. 6B, reconfiguration can be enacted by withdrawal of biomarker luminescent material fluid from lumen **604** of needle **602** via fluid extraction port **618,** which can be in fluidic communication with the lumen of the needle via extraction holes **620.** Extraction holes **620** can be formed by any suitable process (conventional machining, laser drilling, etching, and so on). Withdrawal of biomarker luminescent material fluid is indicated by arrows **622,** which indicate the direction of biomarker luminescent material fluid flow during withdrawal. Semi-permeable barrier 606 can be slidably configured in lumen **604** of needle **602.** As biomarker luminescent material fluid is withdrawn from lumen **604,** a slidable semi-permeable barrier **606** can be drawn in the proximal direction (toward the right of FIGS. 6A and 6B) from its prior distal position (at tip **608** of needle **602).** Semi-permeable barrier **606** is illustrated at a proximal end of lumen **604,** after substantially complete withdrawal of biomarker luminescent material fluid from the lumen. In some alternative examples, semi-permeable barrier **606** could take the form of a non-slidable burst-barrier. It may be generally desirable for such a burst-barrier not to generate any particulates upon bursting.

With biomarker luminescent material fluid withdrawn from lumen **604** of needle **602,** system **600** can be used for delivery of fluid from a fluid delivery system (not illustrated) via fluid input port **624,** which can be in fluidic communication with the lumen of the needle via delivery holes **626.** Delivery holes **626** can be formed by any suitable process (conventional machining, laser drilling, etching, and so on). Delivery of a fluid from a fluid delivery system is indicated by arrows **628.**

Note that space **630** in the cross-sectional view of FIGS. 6A and 6B can be in fluidic communication with fluid input port **624,** for example via an annular space that surrounds needle **602.** Similarly, space **632** can be in fluidic communication with fluid extraction port **618.**

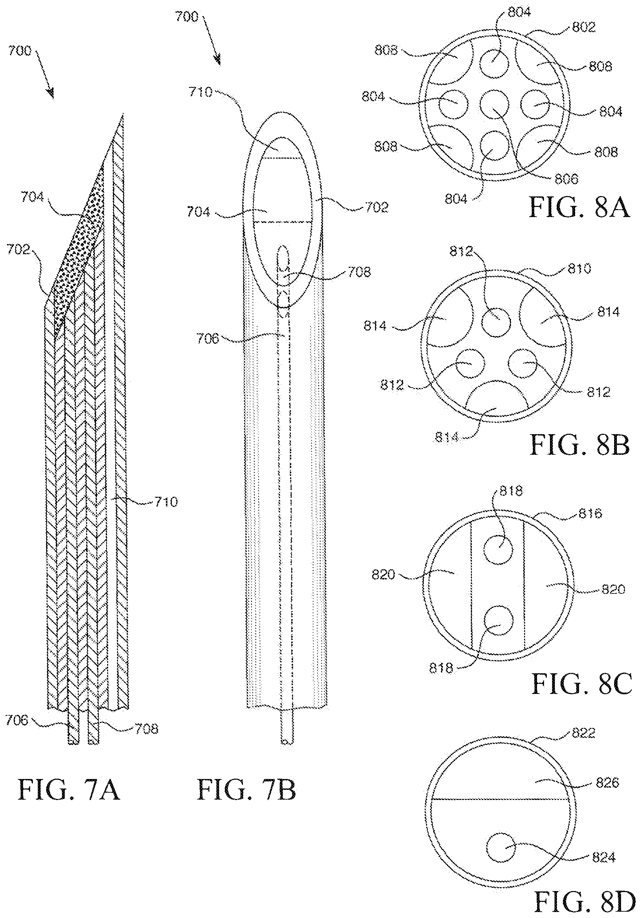

Other needle configurations are possible for biomarker detection via sensing luminescent emissions produced as a result of contact between a biomarker luminescent material and a target biomaterial. FIG. 7A is a schematic cross-sectional view of a biomarker detection needle **700** and FIG. 7B is a schematic plan view of needle **700** from a viewpoint at the left of the needle in FIG. 7A. At its tip **702,** needle **700** can include a biomarker luminescent material **704.** Needle **700** can include one or more optical fibers **706, 708.** One of optical fibers **706, 708** can be used to deliver illumination light from light source (not illustrated), such as a diode laser or other suitable light source, to biomarker luminescent material **704,** and the other of the two optical fibers can be used to transport light emitted from the biomarker luminescent material to an optical receiver (not illustrated), which can be a photo detector or any other suitable light detecting device. Needle **700** can include a lumen **710** suitable for fluid delivery from a syringe or other suitable fluid dispensing system (not illustrated).

The configuration of needle employs non-retracting optical fibers **706, 708** for high-efficiency transport of illumination light and light emitted by the biomarker luminescent material, and simultaneously provides a lumen that is always open for fluid delivery to the tip **702** of the needle. In comparison, in system **200** of FIG. 2, retraction of optical fiber **212** from tip **206** to location **216** may be needed to open lumen **210** for fluid delivery. In some other examples of the present disclosure, such as system **100** of FIG. 1, the open lumens of needles may be used to provide optical waveguides for light transportation, without an optical fiber or fibers extending to the distal tip of said needles. In many instances, optical fibers can provide higher efficiency transport of light than the lumen of a needle.

FIGS. 8A, 8B, 8C, and 8D are schematic cross-sectional views down the bores of needles that provide optical fibers and lumens along their lengths to the needles' tips, similar to needle **700** of FIGS. 7A and 7B. Needle **802** of FIG. 8A can include five optical fibers, with outer fibers **804** being illumination fibers delivering illumination light from a light source to a biomarker luminescent material and inner fiber **806** being a sensing or detection fiber used to transport light emitted from the biomarker luminescent material to an optical receiver. This arrangement of four outer illumination fibers and one inner detection fiber is just an example and should not be considered limiting. Other fiber arrangements are contemplated. Needle **802** can include one or more lumens **808** suitable for fluid delivery. In some examples, multiple lumens can be employed to provide higher fluid conductance for fluid delivery from a common fluid reservoir. In some other examples, multiple lumens can be employed to provide independent delivery paths for different fluids.

Needle **810** of FIG. 8B can include three optical fibers **812** and three lumens **814** suitable for fluid delivery. Needle **816** of FIG. 8C can include two optical fibers **818** and two lumens **820.** Needle **822** of FIG. 8D can include single optical fiber **824** and single lumen **826.** These are just examples, and other quantities of optical fibers and lumens can be provided and employed in biomarker detection needles contemplated in the present disclosure. An optical fiber in a needle having a single optical fiber can be employed for both illumination and detection by using, for example, a fiber optic splitter (similar to splitter **222** of system **200** of FIG. 2) for handling coupling of illumination light and detection light with the single fiber. Alternately, an optical fiber in a needle having a single optical fiber can be employed only for transporting detection light from a biomarker luminescent material to an optical receiver in detection arrangements that do not require illumination light.

In some examples contemplated in the present disclosure, instruments with multiple optical fibers, similar to (but not limited to) the needles illustrated in FIGS. 7A, 7B, 8A, 8B, and 8C can be used for devices configured for detection of multiple biomarkers and/or other detectable substances. Each of multiple fibers can be used for independent detection and/or illumination channels. For example, different biomarker luminescent materials can be coated at the ends of different detection fibers at a needle tip, such that different luminescent detection signals can be sensed independently. Different biomarker luminescent materials may have different illumination requirements, which can be provided by multiple illumination fibers. As discussed elsewhere herein, a separate fiber can be employed for air/gas detection.

Biomarker luminescent materials used for biomarker detection in systems and methods of the present disclosure can exploit various different luminescent phenomena. Some biomarker luminescent materials can rely upon chemiluminescence, a chemical reaction that can occur upon contact between a biomarker luminescent material and target biomarker can, without additional energy input, result in light emission that can be sensed as a signal of detection of the biomarker. Systems **400** and **600,** which do not necessarily include a light source, may be particularly suited for use with chemiluminescent biomarker luminescent materials, given that they may be less complex (at least in optical complexity) than systems that do include light sources. However, potentially any of systems **100, 200, 300, 400, 500,** and **600,** and any of needles **700, 802, 810, 816,** and **822,** could be used in conjunction with chemiluminescent detection, although the inclusion of light sources in some of said systems may be irrelevant to detection of light produced by chemiluminescence.

Some other biomarker luminescent materials can employ photoluminescence (e.g., fluorescence and/or phosphorescence) that is activated by contact between a biomarker luminescent material and target biomaterial. Illumination light for photoluminescence can be provided by light sources of illustrative example systems **100, 200, 300,** and **500,** and can be transported by optical fibers (and/or in some cases, other waveguides) of systems **100, 200, 300, 500,** and at least some of needles **700, 802, 810, 816,** and **822.**

Some biomarker luminescent materials may exhibit photoluminescence in the absence of a target biomarker, and upon exposure to the target biomarker, the photoluminescence can cease or reduce.

Physical characteristics of biomarker luminescent material coatings can reflect a balance between competing factors. A thin coating can be translucent enough to allow illumination light to penetrate in, and emissions to escape for detection, while a thicker coating can provide greater biomarker detection material and a stronger emission signal. Coatings can include cross-linked hydrophilic coatings. The cross-linked hydrophilic coatings can include the biomarker luminescent material as a part of the coating or can encapsulate or seal it to the delivery device. Porosity of the biomarker luminescent material may be desirable to facilitate interaction between biomarkers and the material.

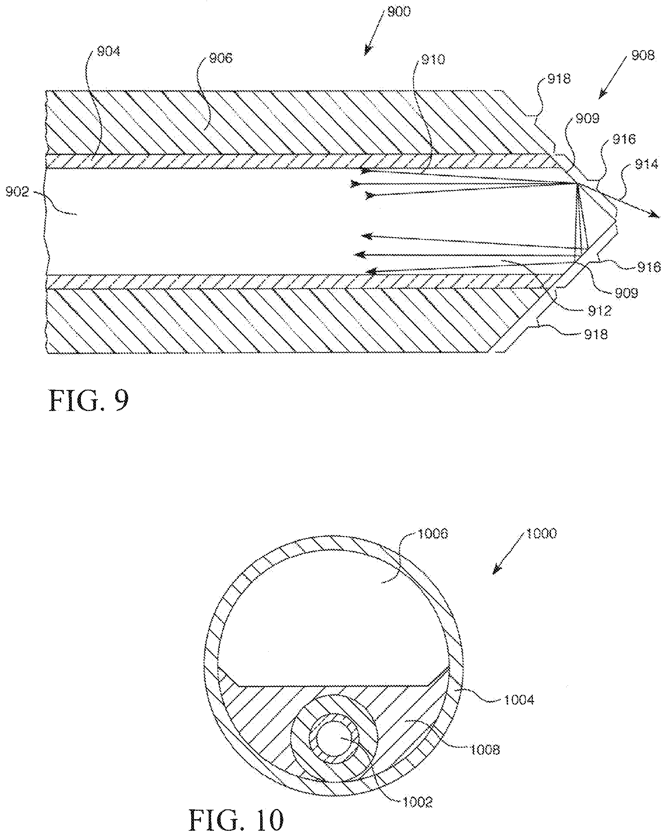

FIG. 9 is a schematic cross-sectional diagram of a fiber optic sensor **900** for distinguishing between air and liquid (which may be referred-to herein as a "fiber optic air sensor"). Sensor **900** can include fiber optic core **902,** which can be surrounded by cladding **904,** which in turn can be surrounded by buffer **906.** Buffer **906** can include one or more buffer layers, such as a primary buffer layer and a secondary buffer layer. Fiber optic air sensor **900** can include any other suitable layers (not illustrated) for strength, protection, etc.

Fiber optic air sensor **900** can be structured and configured to distinguish between liquid and air at a detection end **908** based upon whether light from a light source (not shown) propagating within the fiber toward the detection end (i.e., from left to right in FIG. 9) experiences total internal reflection upon incidence upon faces **909** of fiber core **902** at the detection end. An example bundle of light rays are illustrated as propagating within the core **902** toward (at **910)** detection end **908,** and then, after reflecting off faces **909,** propagating away (at **912)** from the detection end. In the case of total internal reflection, rays of light that are incident upon faces **909** at angles of incidence shallower than the critical angle for total internal reflection can be essentially completely reflected. If incident at an angle steeper than the critical angle, then in general a ray can be partially reflected within the core **902** (as at **912)** and partially refracted out of the fiber, as illustrated schematically for ray **914.**

Faces **909** can be structured to retroreflect rays of light propagating within core **902** toward detection end **908.** They can, for example, be oriented at 45 degrees with respect to the longitudinal axis of core **902.** In some embodiments, they can be arranged in a cube corner configuration. Faces oriented at 45 degrees with respect to the longitudinal axis (which is essentially the light propagation axis) of core **902** can be suitably oriented for discrimination between air and liquid. For a fused silica fiber, the critical angle for total internal reflection relative to an external medium of air is approximately 43 degrees, and the critical angle for total internal reflection relative to an external medium of water is approximately 67 degrees. Therefore, light propagating along the longitudinal axis of core **902** and incident upon a face **909** that is oriented at 45 degrees with respect to the longitudinal axis can be incident upon the face at shallower than the critical angle for air and steeper than the critical angle for water.

In operation, a detection scheme can include an optical receiver (not illustrated) that can be suitably configured to detect light from the light source that has retroreflected from detection end **908.** This retroreflection signal generally can be brighter when faces **909** of detection end **908** are exposed to an external medium of air, resulting in total internal reflection, as opposed to when the faces are exposed to liquid (and hence not resulting in total internal reflection). Faces **909** can include coatings to enhance their detection utility. Inner portions **916** of faces **909,** including the portions of the faces where light in the core can be incident, can have a hydrophobic coating, to repel residual liquid on the faces when the detection end **908** is in air. Outer portions **918** of faces **909** can include a hydrophilic coating to draw liquid away from the inner portions **916.**

FIG. 10 is a schematic cross-sectional view down the bore of an illustrative example of a needle system **1000** that can incorporate a fiber optic air sensor **1002.** Sensor **1002,** which can be like fiber optic air sensor **900** of FIG. 9, can be disposed within hypodermic needle **1004** within mold **1008.** Hypodermic needle **1004** can enclose fluid channel **1006** for delivery of medicinal fluids, but this is not limiting, and in other embodiments the needle can deliver or house other therapeutic payloads and/or devices. It is contemplated that fiber optic air sensors can be incorporated into other configurations of medical devices, including in combination with biomarker detection systems as described herein.

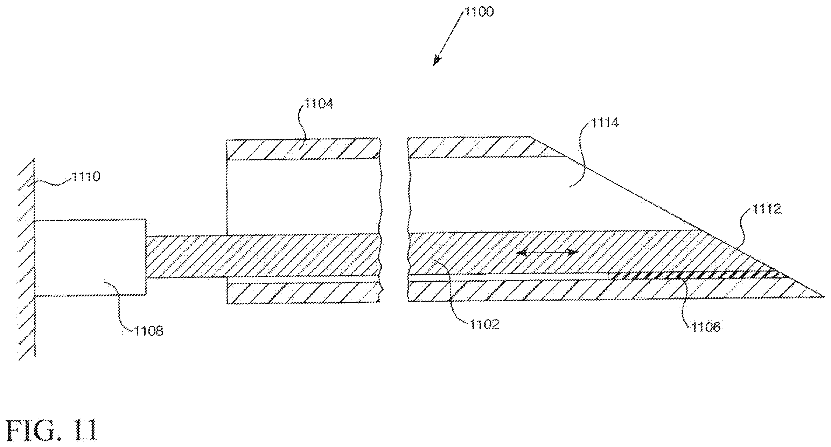

FIG. 11 is a schematic cross-sectional view of needle system **1100** that can incorporate a sonic probe for distinguishing between air and liquid (which may be referred-to herein as a "sonic air sensor"). Sensing rod **1102** can be mounted within hypodermic needle **1104** via one or more elastomeric attachments **1106.** Sensing rod **1102** can be driven longitudinally (as indicated by the arrow superimposed thereupon) by piezoelectric actuator **1108** relative to a reaction mass **1110** at an appropriate frequency. Tip **1112** of sensing rod **1102** at the distal end of needle system **1100** can be in mechanical contact with whatever medium may exist at its location, whether tissue, liquid, or gas. Each of these media can present a different mechanical impedance to the motion of sensing rod **1102,** with impedance generally decreasing in order (tissue>liquid>gas). Sonic/mechanical impedance can be measured in a variety of ways, including, but not limited to, (a) apply constant drive force and measure amplitude; (b) drive to constant amplitude and measure drive force; and/or (c) measure the phase shift between drive force and motion. Needle system **1100** can enclose a fluid channel **1114** for delivery of medicinal fluids, but this is not limiting, and in other examples the needle can deliver or house other therapeutic payloads and/or devices. It is contemplated that sonic air sensors can be incorporated into other configurations of medical devices, including in combination with biomarker detection systems as described herein.

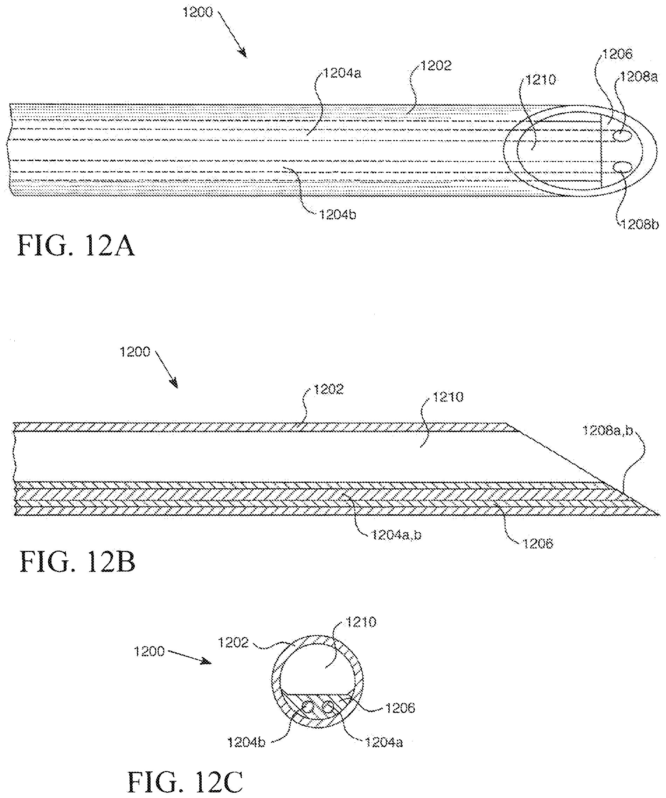

FIGS. 12A, 12B, and 12C are, respectively, a schematic plan view, a schematic side cross-sectional view, and a schematic cross-sectional view down the bore of an illustrative example of a needle system **1200** that can incorporate an electrical sensor for distinguishing between air and liquid (which may be referred-to herein as an "electrical air sensor"). Conductors **1204a, 1204b** can be molded in insulator **1206** residing within hypodermic needle **1202.** Hypodermic needle **1202** can be grounded and conductors **1204a, 1204b** can be connected to an electrical driving and sensing apparatus (not illustrated). The ends of conductors **1204a, 1204b** can be polished at the distal tip of needle **1202** such that their faces **1208a, 1208b** can be in conductive contact with whatever medium is at the tip of the needle. In general, the electrical conductivity between faces **1208a** and **1208b** of conductors **1204a, 1204b** can depend on said medium. For example, the conductivities [measured in (ohm•cm)⁻¹] of human blood plasma (13.5x10⁻³) is significantly different from that of gastric juice (24x10⁻³) and urine (40x10⁻³). The conductivity of air would generally be significantly lower. A hydrophobic coating can be placed at the end of the needle to aid in rejection of liquid from the faces **1208a** and **1208b** of conductors **1204a, 1204b** upon encountering an air space. By monitoring the conductivity between the faces **1208a** and **1208b** of conductors **1204a, 1204b,** differences in media present at the tip of needle system **1200** can be detected, and in some cases, identified. Needle system **1200** can enclose a fluid channel **1210** for delivery of medicinal fluids, but this is not limiting, and in other examples the needle can deliver or house other therapeutic payloads and/or devices. It is contemplated that electrical air sensors can be incorporated into other configurations of medical devices, including in combination with biomarker detection systems as described herein.

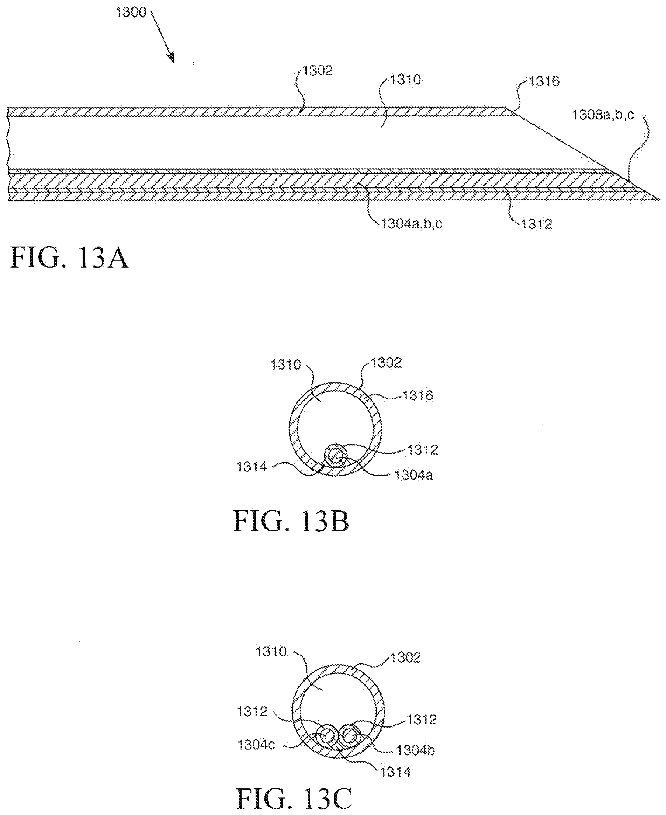

Needle systems similar to system **1200** of FIGS. 12A, 12B, and 12C are illustrated in FIGS. 13A, 13B, and 13C, which are, respectively, a schematic side cross-sectional view of a illustrative example of a needle system **1300** that can incorporate an electrical air sensor, and schematic cross-sectional views down the bores of two alternative configurations of needle system **1300.** In the configurations of FIGS. 13A, 13B, and 13C, wires can be bonded within the lumens of hypodermic needle **1302,** as compared with needle system **1200,** in which conductors **1204a, 1204b** are molded within insulator **1206** within the lumen of needle **1202.** Wires of the configurations of FIGS. 13A, 13B, and 13C each include a conductor **1304a, 1304b,** or **1304c,** with each conductor surrounded by insulator **1312.** The wires can be bonded within the needle lumen with adhesive **1314.**

In the configuration of FIG. 13B, a single wire with conductor **1304a** can be bonded within needle **1302.** In this configuration, conductor **1304a** serves as one conductor, and needle **1302** serves as the other conductor for electrical air sensing. In the configuration of FIG. 13C, two wires having conductors **1304b, 1304c** can be bonded within needle **1302** and serve as the two conductors needed to complete the air sensing circuit. A hydrophobic coating can be placed at the end of the needle to aid in rejection of liquid from the faces **1308a, 1308b, 1308c** of conductors **1304a, 1304b, 1304c,** and the end **1316** of needle **1302,** upon encountering an air space. Needle system **1300** can enclose fluid channel **1310** for delivery of medicinal fluids, but this is not limiting, and in other examples the needle can deliver or house other therapeutic payloads and/or devices.

In an example method of use of a needle system having an optical, sonic, or electrical air-detection system, such as one of systems **1000, 1100, 1200,** or **1300,** the needle can be advanced into a patient by a clinician, with the air-detection system activated to provide feedback to the clinician. Upon advancement of the tip of the needle into a media of interest, a notification system (not illustrated) operatively coupled to the air-detection system can inform the clinician that the target media has been detected. The clinician can then position the tip of the needle in accordance with the detection of the target media and knowledge of the patient's anatomy (for example, further advancing, stopping advancing, or retracting the needle). With the tip of the needle appropriately placed, delivery of a therapeutic fluid from the fluid delivery system (or other therapeutic action) can be performed.

The present disclosure further contemplates real-time systems and methods for distinguishing between gas (which may be referred to as "air") and liquid within patients' anatomies to assist in the precise placement of surgical instruments therein. Such systems can include optical, sonic, and/or electrical detection, and can be based upon differences in optical, sonic, and/or electrical impedance.

The present disclosure also contemplates real-time systems and methods for determining the location of a biomarker detection system that can detect the presence of a target biomarker in real-time. In some examples, disclosed biomarker detection system can include a probe that can sense the presence of specific types of bodily tissues. For example, a biomarker detection system according to the present disclosure can include a probe that detects iron and thus the presence of blood when in contact with that type of tissue. The disclosed real-time probe for blood can signal whether or not it is in close proximity to blood-signaling the presence of blood when the probe is in contact with blood and the absence of blood when the probe is no longer in contact with blood. Real-time probes according to this disclosure can be useful, for example, in endoscopic procedures that involve probing inside of the body during surgery or in trying to introduce an anesthetic agent in a tight location such as the spinal cavity as discussed above. Such a probe can operate by detecting a concentration of iron in proximity to the probe.

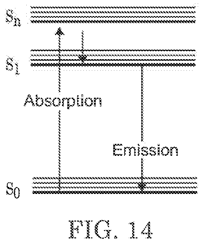

A simplified, typical fluorescence process is shown in the Jablonski diagram illustrated in FIG 14. Fluorescence is defined as "the molecular absorption of light energy (photon) at one wavelength and its re-emission at another, longer, wavelength." During that process a photon can be absorbed from, for example, a ground state (S₀) of a fluorescent molecule bumping it up into an excited state such as an excited state (Sₙ) of the energy gap between the ground state and the excited state matches the energy of the absorbed radiation. Frequently, as shown in FIG. 14 the electron can be promoted to an upper excited state such as (Sₙ) in which the excited molecule can lose energy through vibrational and conformation changes until it reaches its lowest excited state (S₁). When the (S₁) state is populated with enough electrons, the excited state of the fluorescent molecule can cascade back to ground state (S₀) emitting a photon of a different wavelength than that absorbed. The emitted photon is almost always of a lower energy (higher wavelength) than the absorbance event. The fluorescence process is cyclical therefore a fluorophore can be excited repeatedly Fluorescence Emission Absorption of a photon and thus excitation to (S1) or (Sn) respectively. Reconversion to (S₀) from (S₁) with emission of radiation (fluorescence).

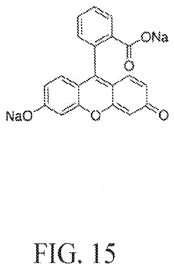

FIG. 15 is a representation of the chemical structure and formula of the molecule, fluorescein. Fluorescein (C₂₀H₁₀Na₂O₅) and closely related structures can persist and remain actively luminescent (fluorescent) for time periods in excess of a typical medical procedure. The needle detection and luminescence with a fluorescein or fluorescein-like) coating can be persistent and can remain actively luminescent for time in excess of the typical medical procedure. Once iron ions (Fe²⁺) from blood contacts the coating, the iron serves as a catalyst for enabling the reemission of absorbed radiation at a different frequency and is not consumed in a reaction.

The fluorescein molecule has a strong affinity and selectivity to iron ions, having two unshared electron pairs available for donation to a metal ion. Once the covalent bond occurs, the molecule gains the ability to fluoresce. This selectivity for chelating iron ions is one of the reasons that Fluorescein is an ideal choice for this sensor. This sensor platform relies upon photoinduced electron transfer (PET) between this fluorophore and its metal-specific chelate, Fe²⁺. Also, in general, the persistence of the fluoresce property is a benefit. In a chemical process where the iron is consumed, such as with luminol, the persistence of the luminescence is limited to the time in which the iron is being consumed, often shorter than a surgical procedure. The intensity of the lamination also decreases over time.

The problem with relatively unlimited florescence time is that when leaving an iron ion rich environment, the sensor remains in the "on" state. If the goal of one embodiment of the sensor is to make a sensor with real-time dynamic response to iron ion concentration, then the key objective is to break these iron ion bonds at a steady rate such that the fluorescence reduces over time as the sensor passes to tissue with lower iron concentration.

The present embodiment designed to measure real-time iron concentration can involve a means to steadily draw the iron ions away from the florescence media coating from the backside of the coating, while the frontside of the florescence media coating is exposed to the sample fluid. The present disclosure introduces a biomarker detection probe that utilizes a second coating underneath the florescence media coating with a stronger affinity for the iron ions. The florescence media coating can be a semi permeable membrane such that the iron ion concentration in this coating creates florescence when the front-side of the coating experiences high iron concentrations and is swept clean of iron ions when the front-side of the coating is exposed to a low iron ion concentration, reducing the intensity of the florescence accordingly. The composition of this second layer can consist of many different materials with the desired properties to clear ions from the detection layer over time.

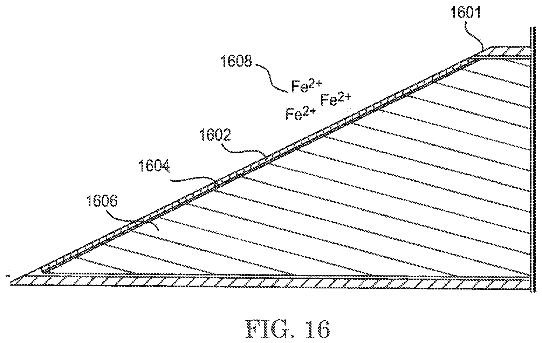

FIG. 16 is planar side cross-section illustration of an embodiment of a real-time fluorescent probe. The real-time fluorescent probe shown in FIG. 16 is incased within the diameter of needle **1601.** Needle **1601** has fluorescent coating **1602** (containing, for example, fluorescein or a related molecule) on its outside edge that is in contact with biological tissue when needle **1601** is inserted into a biological system. Fluorescent coating **1602** is disposed upon ion-consuming coating **1604** that effectively removes iron ions after detection. Fluorescent coating **1602** with ion-consuming coating **1604** disposed thereupon is adjacent to fiber optic waveguide **1606** that can conduct radiation emitted from fluorescent coating **1602** to a detector when fluorescent coating **1602** is in the presence of iron ions **1608.**

Coating thicknesses and permeability of the different layers can be adjusted to modify the ion sensing response time. Coating compositions must be substantially transparent to the light wavelength emitted by the florescent coating so that emitted light will enter the fiber optic wave guide or selected optical transmission medium.

Another contemplated example can incorporate nanoparticles in the florescence media coating itself wherein the fluorescent media coating can slowly release chemicals to consume the iron ions over time, but not so fast as to inhibit the florescent coating from illuminating in the presence of the target ion. Again, the release rate can be adjusted to modify the ion sensing response time.

## Claims

1. A probe for real-time sensing of a target biomarker comprising:
a needle (1601) comprising a tip and having an opening;
a luminescent probe encased within the opening of the needle,
wherein the luminescent probe comprises a biomarker luminescent material disposed within and covering the opening of the needle;
a coating (1602) comprising the biomarker luminescent material adjacent to or inside the tip of the needle;
wherein the biomarker luminescent material is in contact with biological tissue; and **characterised by**
a first ion-consuming coating (1604) within the needle and adjacent to the coating.

2. The probe for real-time sensing of a target biomarker according to claim 1,
wherein the biomarker luminescent material comprises a fluorescent molecule, and
wherein, optionally, the fluorescent molecule comprises fluorescein.

3. The probe for real-time sensing of a target biomarker according to claim 1, wherein the first ion-consuming coating detects iron and the presence of blood.

4. The probe for real-time sensing of a target biomarker according to claim 3, wherein the probe can signal the presence or absence of blood in real-time.

5. The probe for real-time sensing of a target biomarker according to claim 3, further comprising a second ion-consuming coating disposed underneath the first ion-consuming coating wherein the second ion- consuming coating has a stronger affinity for iron than the first ion-consuming coating.

## Patentansprüche

1. Sonde zur Echtzeiterfassung eines Ziel-Biomarkers, Folgendes umfassend:
eine Nadel (1601), umfassend eine Spitze und eine Öffnung;
eine lumineszierende Sonde, die innerhalb der Öffnung der Nadel eingeschlossen ist, wobei die lumineszierende Sonde ein lumineszierendes Biomarkermaterial umfasst, das in der Öffnung der Nadel angeordnet ist und diese bedeckt;
eine Beschichtung (1602), die das lumineszierende Biomarkermaterial neben oder innerhalb der Nadelspitze umfasst;
wobei das Biomarker-Lumineszenzmaterial in Kontakt mit biologischem Gewebe steht; und durch eine erste ionenverbrauchende Beschichtung (1604) innerhalb der Nadel und angrenzend an die Beschichtung gekennzeichnet ist.

2. Sonde zur Echtzeiterfassung eines Ziel-Biomarkers nach Anspruch 1,
wobei das Biomarker-Lumineszenzmaterial ein fluoreszierendes Molekül umfasst und
wobei das fluoreszierende Molekül optional Fluorescein umfasst.

3. Sonde zur Echtzeiterfassung eines Ziel-Biomarkers nach Anspruch 1, wobei die erste ionenverbrauchende Beschichtung Eisen und das Vorhandensein von Blut erkennt.

4. Sonde zur Echtzeiterfassung eines Ziel-Biomarkers nach Anspruch 3, wobei die Sonde das Vorhandensein oder Nichtvorhandensein von Blut in Echtzeit signalisieren kann.

5. Sonde zur Echtzeiterfassung eines Ziel-Biomarkers nach Anspruch 3, ferner umfassend eine zweite ionenverbrauchende Beschichtung, die unter der ersten ionenverbrauchenden Beschichtung angeordnet ist, wobei die zweite ionenverbrauchende Beschichtung eine stärkere Affinität für Eisen aufweist als die erste ionenverbrauchende Beschichtung.

## Revendications

1. Sonde de détection en temps réel d'un biomarqueur cible comprenant :
une aiguille (1601) comprenant une pointe et présentant une ouverture ;
une sonde luminescente enfermée à l'intérieur de l'ouverture de l'aiguille, dans laquelle la sonde luminescente comprend un matériau luminescent biomarqueur disposé à l'intérieur et recouvrant l'ouverture de l'aiguille ;
un revêtement (1602) comprenant le matériau luminescent biomarqueur adjacent à ou à l'intérieur de la pointe de l'aiguille ;
dans laquelle le matériau luminescent biomarqueur est en contact avec un tissu biologique ; et **caractérisé par** un premier revêtement consommateur d'ions (1604) à l'intérieur de l'aiguille et adjacent au revêtement.

2. Sonde de détection en temps réel d'un biomarqueur cible selon la revendication 1,
dans laquelle le matériau luminescent biomarqueur comprend une molécule fluorescente, et
dans laquelle, éventuellement, la molécule fluorescente comprend de la fluorescéine.

3. Sonde de détection en temps réel d'un biomarqueur cible selon la revendication 1, dans laquelle le premier revêtement consommateur d'ions détecte le fer et la présence de sang.

4. Sonde de détection en temps réel d'un biomarqueur cible selon la revendication 3, dans laquelle la sonde peut signaler la présence ou l'absence de sang en temps réel.

5. Sonde de détection en temps réel d'un biomarqueur cible selon la revendication 3, comprenant également un second revêtement consommateur d'ions disposé sous le premier revêtement consommateur d'ions, dans laquelle le second revêtement consommateur d'ions présente une affinité plus forte pour le fer que le premier revêtement consommateur d'ions.
